# EUROPEAN PATENT APPLICATION

(11) **EP 4 361 286 A1**
(43) Date of publication of application: **01.05.2024**
(21) Application number: 22203474.6
(22) Date of filing: 25.10.2022
(51) Int. Cl.: C12Q 1/6841

(54) **METHOD FOR DETERMINING THE SPATIAL LOCALISATION OF RNA STRANDS ON A SURFACE**

(71) Applicant: Miltenyi Biotec B.V. & Co. KG, 51429 Bergisch Gladbach (DE)
(72) Inventor: PEROBST, Michel, 51429 Bergisch Gladbach (DE); PINARD, Robert, 51429 Bergisch Gladbach (DE); HOSONO, Seiyu, 51429 Bergisch Gladbach (DE)
(74) Representative: Kisters, Michael Marcus

(57) **Abstract**

The invention is directed to a method for determining the spatial localisation of at least one RNA strand on a surface comprising the steps
a. hybridizing at least one oligonucleotide comprising 50 - 1000 nucleotides of which 5 to 20 nucleotides serve as detection sequence; with its 5' and 3' ends to complementary parts of the at least one RNA strand
b. combining the 5' and 3' ends of the at least one oligonucleotide to create a single strand circular template hybridized to the at least one RNA strand
c. multiplying the single strand circular template by a polymerase capable of rolling circle amplification into a plurality of concatemers thereby forming a rolony
**characterized in**
d. providing a plurality of detection probes comprising one fluorescence dye and a detection oligonucleotide which is at least in part complementary to the detection sequences in the rolonies and hybridizing the detection probes to the detection sequences in the rolonies
e. removing unhybridized detection probes
f. imaging the rolonies, thereby determining the spatial localisation of the RNA strands on the surface.

## Description

### Field of invention

The present invention relates to a method for determining the spatial localisation of at least one RNA strand on a surface utilizing amplification of padlock oligonucleotides hybridized to the RNA strand.

### Background

Padlock oligonucleotides have proven to be very successful in polymerizing short portion of nucleic acids to which it has been hybridized to. Most padlock approaches begin by reverse transcribing the target into cDNA.

Padlock methods are for example disclosed in "Highly multiplexed subcellular RNA sequencing in situ" by Lee et al., Science. 2014 March 21; 343(6177): 1360-1363. doi:10.1126/science.1250212 or "Efficient In Situ Detection of mRNAs using the Chlorella virus DNA ligase for Padlock Probe Ligation" by Nils Schneider and Matthias Meier; February 5, 2020 -Cold Spring Harbor Laboratory Press

A comprehensive assay for targeted multiplex amplification of human DNA sequences is published by Sujatha Krishnakumar et al.; PNAS sent for review February 19, 2008.

Further, WO2017143155A2 discloses multiplex alteration of cells using a pooled nucleic acid library and analysis thereof and WO2018045181A1 discloses Methods of generating libraries of nucleic acid sequences for detection via fluorescent in situ sequencing

The published Padlock methods allow sequencing of DNA or RNA, but do not give any information from what specific cell or tissue location the sequenced DNA or RNA origins from.

Microscopy imaging that allow for multiple mRNAs to be resolved at a single cell level provides valuable information regarding transcript amount and localization, which is a crucial factor for understanding tissue heterogeneity, the molecular development and treatment of diseases.

However, besides sequencing, the mere location of RNA on a tissue is of interest. Since RNA is a rather instable molecule, there was a need for a method which is able to determine the special location of a RNA molecule in a quick and robust manner.

### Summary of the invention

Object of the invention is a method for determining the spatial localisation of at least one RNA strand on a surface comprising the steps
a. hybridizing at least one oligonucleotide comprising 50 - 1000 nucleotides of which 5 to 20 nucleotides serve as detection sequence; with its 5' and 3' ends to complementary parts of the at least one RNA strand
b. combining the 5' and 3' ends of the at least one oligonucleotide to create a single strand circular template hybridized to the at least one RNA strand
c. multiplying the single strand circular template by a polymerase capable of rolling circle amplification into a plurality of concatemers thereby forming a rolony
**characterized in**
d. providing a plurality of detection probes comprising one fluorescence dye and a detection oligonucleotide which is at least in part complementary to the detection sequences in the rolonies and hybridizing the detection probes to the detection sequences in the rolonies
e. removing unhybridized detection probes
f. imaging the rolonies, thereby determining the spatial localisation of the RNA strands on the surface.

The method takes advantage of the amplification of the detected signal by multiplication of the detection sequence by RCA.

Tn a preferred embodiment, the rolonies are crosslinked before imaging. The crosslinking agents used in the present invention may be those known from the art of oligonucleotide capture on solid surface or tissue, e.g.,microarray generation, protein:protein interactions, isolating cell surface proteins and preparing labeled probes.

### Detailed description

The present method combines the use of oligonucleotide forming a padlock while hybridized (padlock probes) and their sequential hybridization for the detection of bound probes.

The padlock may be created in a first embodiment with a gap-fill step. In this embodiment, the oligonucleotide is hybridized with its 5' and 3' ends to such complementary parts of the at least one RNA strand that a gap is created between the 5' and 3' ends of the oligonucleotide and wherein this gap is filled with complementary nucleotides using the RNA strand as template.

The gap-fill step is performed with a reverse polymerase filling the open section between the hybridized 3' and 5' portion of the oligonucleotide from a circular molecule.

In another embodiment, the padlock is created without a gap fill step by ligating the 3' and 5' with each other. Here, the oligonucleotide is hybridized with its 5' and 3' ends to such complementary parts of the at least one RNA strand that the 5' and 3' ends of the oligonucleotide are hybridized adjacent to each other and wherein 5' and 3' ends of the oligonucleotide are ligated with each other.

### Detection probes

The detection probes used in the method of the present invention may be comprised of a) a oligonucleotide with 2 to 20 nucleotides capable of binding to at least a part of the barcode region and b) a detection region selected from the group consisting of a magnetic particle, a fluorescence dye, and a radioactive label.

Preferable, the detection probes comprise fluorescent dyes known from the art of immunofluorescence technologies, e.g., flow cytometry or fluorescence microscopy. Useful fluorescent moieties might be protein-based, such as phycobiliproteins, polymeric, such as polyfluorenes, small organic molecule dyes, such as xanthenes, like fluorescein, or rhodamines, cyanines, oxazines, coumarins, acridines, oxadiazoles, pyrenes, pyromethenes, or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties.

Further, the detection region may comprise a radioactive label, for example in the form of radioisotope labeling by exchanging non-radioactive isotopes for their radioactive counterparts, such as tritium, 32P, 35S or 14C, or introducing covalently bound labels, such as 1251, which is bound to tyrosine, 18F within fluorodeoxyglucose, or metallo-organic complexes, i.e. 99Tc-DTPA.

### Padlock oligonucleotides

In the method of the invention, the padlock oligonucleotides are circularized and the single strand circular template that is generated is replicated by a polymerase capable of rolling circle amplification into a plurality of DNA concatemers forming a DNA nanoball or rolony. For this purpose, the oligonucleotide used in the present invention may comprise at least one primer region with 5 to 50 nucleotides for the rolling circle amplification.

In one embodiment of the invention, the at least one oligonucleotide is provided with at least one primer region for RCA, preferable the least one primer region is located between the barcode region and the 5' and/or the 3' ends of the oligonucleotide.

In another embodiment, the at least one oligonucleotide is provided with at least one sequencing region and that the rolonies are sequenced after imaging.

In further embodiments, the detection probes can be removed from the rolonies for another round of detection. This can be accomplished either by completely removing the hybridized detection probes from the rolonies after imaging the rolonies.

In another variant, only the fluorescence dye is removed from the detection oligonucleotide after imaging the rolonies,.

In yet another embodiment, the method of the invention is capable of multiplexing by providing a plurality of oligonucleotides comprising different detection sequences.

The padlock probe technology is used on probes that is hybridized to a specific portion of a messenger RNA directly on a section of tissue that can be fixed and permeabilized.

In a variant of this embodiment, the sample which is preferable a solid tissue like a fresh-frozen tissue section is placed on a covered glass slide containing the padlock forming oligonucleotide that is tethered to a bead or to an antibody. The tissue is fixed and imaged for histological purposes and permeabilized to release mRNA that can bind to adjacent padlock probes, allowing for the capture of gene expression information and later on the sequence information.

Samples to be analysed with the disclosed method may originate from any specimen, like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo sapiens). A biological sample may have the form of a tissues slice, cell aggregate, suspension cells, or adherent cells. The cells may be living or dead.

The spatial information of the rolonies i.e. the location of the rolonies on the sample is determined for example by an imaging step. In yet another variant of the method according to the invention, the sample is converted into isolated cells which are then immobilized by trapping in microcavities or by adherence.

Imaging may be performed for example with techniques known as "Multi Epitope Ligand Cartography", "Chip-based Cytometry" or "Multiomyx", described for example, in EP 0810428, EP1181525, EP 1136822 or EP1224472. In this technology, cells are immobilized and contacted with antibodies coupled to fluorescent moiety. The antibodies are recognized by the respective antigens on the biological specimen (for example on a cell surface) and after removing the unbound marker and exciting the fluorescent moieties, the location of the antigen is detected by the fluorescence emission of the fluorescent moieties. In certain variants, instead of antibodies coupled to fluorescent moieties, antibodies coupled to moieties detectable for MALDI-Imaging or CyTOF can be used. The person skilled in the art is aware how to modify the technique based on fluorescent moiety to work with these detection moieties. The location of the target moieties is achieved by a digital imaging device with a sufficient resolution and sensitivity for the wavelength of the fluorescence radiation. The digital imaging device may be used with or without optical enlargement for example with a fluorescence microscope. The resulting images are stored on an appropriate storing device like a hard drive, for example in RAW, TIF, JPEG, or HDF5 format.

## Claims

1. A method for determining the spatial localisation of at least one RNA strand on a surface comprising the steps
a. hybridizing at least one oligonucleotide comprising 50 - 1000 nucleotides of which 5 to 20 nucleotides serve as detection sequence; with its 5' and 3' ends to complementary parts of the at least one RNA strand
b. combining the 5' and 3' ends of the at least one oligonucleotide to create a single strand circular template hybridized to the at least one RNA strand
c. multiplying the single strand circular template by a polymerase capable of rolling circle amplification into a plurality of concatemers thereby forming a rolony
**characterized in**
d. providing a plurality of detection probes comprising one fluorescence dye and a detection oligonucleotide which is at least in part complementary to the detection sequences in the rolonies and hybridizing the detection probes to the detection sequences in the rolonies
e. removing unhybridized detection probes
f. imaging the rolonies, thereby determining the spatial localisation of the RNA strands on the surface.

2. Method according to claim 1 **characterized in that** the oligonucleotide is hybridized with its 5' and 3' ends to such complementary parts of the at least one RNA strand that a gap is created between the 5' and 3' ends of the oligonucleotide and wherein this gap is filled with complementary nucleotides using the RNA strand as template.

3. Method according to claim 1 **characterized in that** the oligonucleotide is hybridized with its 5' and 3' ends to such complementary parts of the at least one RNA strand that the 5' and 3' ends of the oligonucleotide are hybridized adjacent to each other and wherein 5' and 3' ends of the oligonucleotide are ligated with each other.

4. Method according to any of the claims 1 to 3 **characterized in that** after imaging the rolonies, the hybridized detection probes are removed from the rolonies.

5. Method according to any of the claims 1 to 3 **characterized in that** after imaging the rolonies, the fluorescence dye is removed from the detection oligonucleotide.

6. Method according to any of the claims 1 to 5 **characterized in** providing a plurality of oligonucleotides comprising different detection sequences.

7. Method according to any of the claims 1 to 6 **characterized in that** the rolonies are crosslinked before imaging.

8. Method according to any of the claims 1 to 7 **characterized in that** the at least one oligonucleotide is provided with at least one primer region for RCA.

9. Method according to any of the claims 1 to 8 **characterized in that** the at least one oligonucleotide is provided with at least one sequencing region and that the arolonies are sequenced after imaging.
